# EUROPEAN PATENT SPECIFICATION

(11) **EP 0 298 694 B1**
(45) Date of publication and mention of the grant of the patent: **06.10.1993**
(21) Application number: 88306104.6
(22) Date of filing: 05.07.1988
(51) Int. Cl.: A61L 2/20, A61L 2/24

(54) **Vapour flow-through systems**
Vorrichtung zum Durchströmen von Dampf
Installation de circulation de vapeur

(30) Priority: 06.07.1987 US 70271
(43) Date of publication of application: 11.01.1989
(73) Proprietor: American Sterilizer Company, Erie, Pennsylvania 16512 (US)
(72) Inventor: Cummings, Arthur L., Tonco City, OK 74604 (US); Childers, Robert W., Erie P.A. 16505 (US); Mielnik, Thaddeus J., Jr., Erie, P.A.16510 (US)
(74) Representative: Jones, Colin

(56) References cited:
- DE-A- 2 313 484
- DE-A- 2 745 961
- DE-A- 3 532 310
- US-A- 4 512 951

## Description

The present invention relates to vapour flow-through systems and is useful for sterilisation systems and more particularly for vapour phase sterilisation systems.

There is often a need to sterilise the interior of a container or a room due to some unexpected contamination or to prepare the container or room for a special use. For example, clean rooms are needed to manufacture certain kinds of microelectronic products and pharmaceuticals. The room must be sterilised periodically. The interior of incubators may require sterilisation to rid them of pathogenic contaminants. In addition, laboratory spills of highly contageous substances may unexpectedly contaminate an area. Such containers and their contents are not easily sterilised by conventional methods.

Gaseous sterilisation systems, such as ethylene oxide, formaldehyde, ozone and hydrogen peroxide, have been used with varying success in a variety of applications. One problem with each of these sterilants is disposing of the residual vapours following sterilisation. See DISINFECTION, STERILISATION, AND PRESERVATION, 592, 677 (S.S. Block 2d ed. 1977).

Conventional gaseous/steam sterilisation systems use vacuum pumps to evacuate chambers prior to introduction of the sterilant. Air blowers and injection pumps, or a vacuum source and gravity feed injection system are also commonly used in such sterilisation systems. Due to the extreme pressure differentials required, vacuum or pressure systems require the use of sturdy, rigorously sealed vacuum/pressure vessels.

One arrangement for a steam-heated autoclave is disclosed in US- A- 3,773,466. The system therein described includes an autoclave, a heating chamber for generating steam, a water tank and a steam trap. Steam entering the autoclave forces air out to the steam trap and to the water tank. At the end of the cycle, steam flows from the autoclave, back through the heating chamber through a three-way valve to the water tank. When pressure within the system is equalized, water flows by gravity from the water tank to the heating chamber for use in subsequent cycles. A related system is disclosed in US- A- 3,443,884.

US- A- 4,169,123, and US- A- 4,169,124, disclose methods of "cold" gas sterilisation using hydrogen peroxide gas at temperatures below 80°C. In US-A- 4,169,123, it is recommended that liquid hydrogen peroxide should be volatilized within the sterilisation chamber but it is also indicated that the volatilization may occur outside of the chamber. The hydrogen peroxide vapour may then be introduced into the sterilization chamber by air displacement. No details are provided as to how the introduction of the vapours by air displacement is to be achieved.

US- A- 4,642,165 discloses a method of vaporizing successive small quantities of a multicomponent liquid, such as an aqueous solution of hydrogen peroxide, for injection into a vacuum chamber. The vacuum in the chamber draws the multicomponent vapour into the chamber.

US- A- 4,512,951, discloses a method of liquid-contact hydrogen peroxide sterilisation. Goods to be sterilised are maintained in the sterilisation chamber at a temperature below the dew point of the vapour sterilant. An aqueous solution of hydrogen peroxide is vaporized and passed into the evacuated sterilisation chamber where, upon contact with the goods, the vapour condenses to form a liquid layer of sterilant on the goods. The vacuum in the chamber draws the vapour in.

GB- A- 1,582,060 discloses a similar liquid contact hydrogen peroxide sterilisation method operated without a vacuum chamber. Liquid hydrogen peroxide is pumped to an ultrasonic spray nozzle which is operated by a stream of dehydrated air. A mist of hydrogen peroxide is sprayed into a container and mixed with hot air to change the mist into a vapour. The vapour is piped into a non-pressurized sterilisation chamber where it condenses on a cool, moving web of material, A stream of hot air in an adjacent chamber removes the hydrogen peroxide layer from the web. The stream is then passed to a water separator where it is relieved of the sterilant.

GB- A- 1,574,488 also discloses a method for removing liquid hydrogen peroxide by means of a hot air stream.

Hydrogen peroxide, although irritating to the skin and eyes, decomposes to water and oxygen. A variety of materials are known which catalytically decompose hydrogen peroxide upon contact. Exotic metal catalysts, such as platinum black, have been evaluated as described in US- A- 3,912,451 for use in removing hydrogen peroxide from contact lenses.

The use of manganese dioxide (MnO₂) supported on alumina in a continuous, tubular, packed bed reactor was evaluated for the catalytic decomposition of hydrogen peroxide by Kohler et al, "Catalytic Decomposition of Hydrogen Peroxide by Manganese-Alumina", NTIS Document PB 80-124274, National Science Foundation, Washington, DC (1974). The MnO₂ does not completely destroy incoming hydrogen peroxide. A second treatment stage employing immobilised catalase is used to destroy any residual hydrogen peroxide.

Other materials which are known to catalyse hydrogen peroxide are metals, such as lead, iron, copper, cobalt, silver, gold and palladium. US- A-4,521,375 discloses the use of pyruvic acid and salts thereof to destroy hydrogen peroxide. It is also known that heat will lead to the decomposition of hydrogen peroxide.

DE-A-2745961 discloses a vapour flow-through system of the kind for use with at least one sealable pressure or non-pressure container and comprising air intake means; a fluid flow path for connecting the air intake means to an inlet to the container; means for withdrawing air from an outlet from the container; at least one reservoir for fluid; and heating means in said fluid flow path.

There is a need for a simple, inexpensive system, preferably in modular form, for use with existing non-pressure or pressure containers or vessels, to generate a vapour, deliver it to the container or vessel and then dispose of the residual vapours.

According to the present invention, a flow-through system of the above kind is characterised in that the reservoir contains a degradable liquid and a means for degrading vapourized liquid to render it suitable for disposal is provided; and in that the reservoir is connected via valve means to the heating means for conducting liquid thereto for heating such liquid to a temperature sufficient to transform the liquid into vapour substantially instantaneously and for introducing it into the air in the fluid flow path, whereby, in operation, a pressure differential is created between the reservoir and the heating means to urge liquid to flow from said reservoir to said heating means when said valve means is open and withdrawal of air from the container reduces the pressure therein sufficiently to draw a stream of air from said air intake means through said heating means where said stream of air carries vapour formed in said heating means through said container inlet into said container, and whereby, in operation, a substantial portion of air and vapour withdrawn from said container by said withdrawing means is directed to said degrading means for degradation and subsequent disposal.

The present invention thus provides a vapour flow-through system which can be integrally associated with sealable containers or which can be a modular unit adapted to releasable connection to a variety of existing pressure or non-pressure sealable containers. The system can be used with at least one sealable container, such as an incubator, a refrigerator, a clean room or any sealable enclosure.

Advantageously, fluid connecting means define a flow-through path from the intake means, through the heating means, into and through the container and past the withdrawing means to a first connection directed to the degrading means. A second connection is preferably directed to the reservoir. The second connection continues from the reservoir, through the valve means to the heating means where the second connection merges with the path from the heating means to the container.

The second connection leading to the reservoir, when employed, is preferably narrower than the first connection to the degrading means so that a substantial portion of air or vapour withdrawn from the container is directed to the degrading means. The remaining portion of air or vapour would then be directed along the narrow second connection to the reservoir to create a pressure differential between the reservoir and the heating means to urge the liquid towards the heating means.

The system may further include a three-way valve disposed along the first connection for selectively directing flow from the container to the degrading means for disposal or to the heating means for recirculation.

There may be two liquid reservoirs, one preferably holding a liquid sterilant, such as an aqueous solution of hydrogen peroxide in suitable concentrations, and the other holding water. The dual reservoir system can be selectively used for sterilisation, or alternatively, for humidification within the container. Each liquid reservoir has associated therewith a respective valve means for controlling delivery of liquid to the heating means. When both valve means are closed, the system can be used for aeration within the container.

When two containers are used, the system further includes an intake three-way valve disposed between the heating means and the containers for selectively directing flow to one or the other container, and an outlet three-way valve disposed between the two containers and the withdrawing means for selectively controlling flow from each container.

The heating means and degrading means are preferably housed in a single unit.

Thus the invention includes apparatus for use with a vapour system comprising:
an inner housing defining an inner chamber having at least one first inlet and a first outlet;
an outer housing surrounding said inner housing to define therebetween an outer chamber having a second inlet and a second outlet;
means within said outer chamber for degrading a degradable vapour to a form suitable for disposal, said degrading means providing sufficient contact with incoming vapour to promote substantially complete degradation; and
heating means for imparting sufficient heat to said outer chamber to enhance degradation of incoming vapour and for providing sufficient heat for said inner chamber to substantially instantaneously transform incoming liquid into vapour.

The degrading means preferably comprise metallic spheres having surfaces for catalytically degrading vapour contacted therewith.

The present invention will be further described, by way of example, with reference to the accompanying drawings in which:-
Fig. 1 is a schematic illustration of one embodiment of a flow-through system of the present invention;
Fig. 2 is a schematic illustration of an alternative embodiment of the system of Fig. 1 with dual liquid reservoirs;
Fig. 3 is a schematic illustration of a third embodiment of the system shown in Fig. 1 with dual containers;
Fig. 4 is a schematic illustration of a fourth embodiment of the system shown in Figs. 2 and 3 with dual liquid reservoirs and dual containers;
Fig. 5 is a section view of the vaporiser/degrader used in the system of the present invention; and
Fig. 6 is a section view of the vaporiser/degrader of Fig. 5 along the live VI-VI.

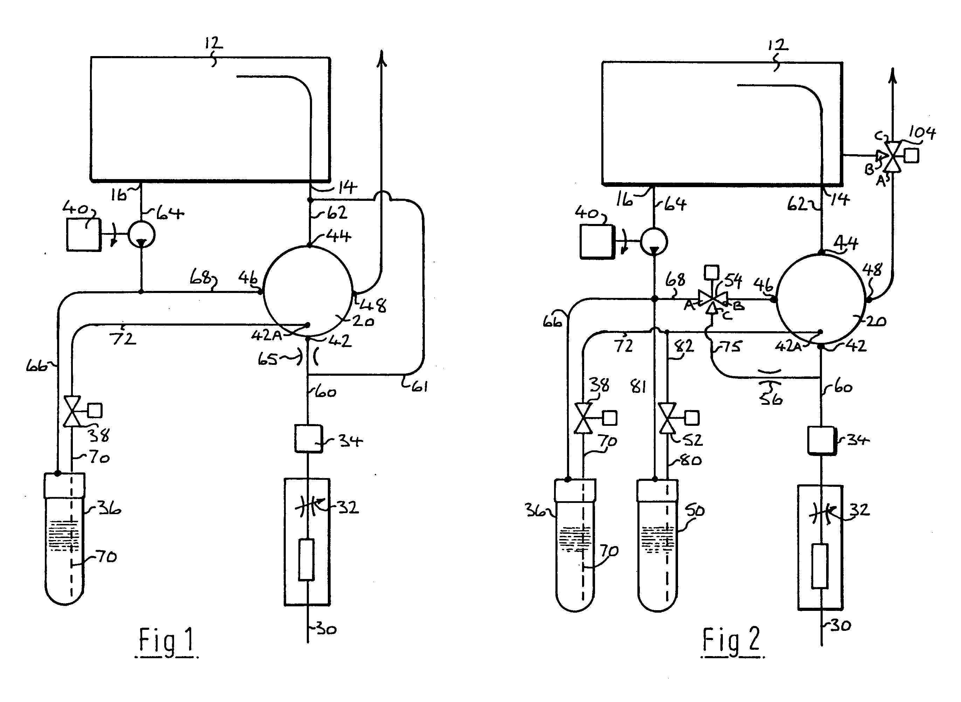

Figs. 1 to 6 illustrate the preferred embodiments of the sterilisation system of the present invention and equipment for use in such a system. The sterilisation system can be housed in modules for releasable or permanent attachment to sealable containers 12. The containers 12 need not be pressure/vacuum vessels and can, therefore, be of weaker construction relative to a typical pressure/vacuum vessel. Any relatively rigid, sealed structure, including, but not limited to, a room, an incubator, a refrigerator or a tent with a rigid frame for placement over an area or a device to be sterilised can provide the container 12 for use with the sterilisation system of the present invention.

Referring to Fig. 1, the sterilisation system, in its simplest form, includes a vaporiser/degrader 20, an air intake 30, an air flow meter 32, air filter 34, liquid sterilant reservoir 36, injection valve 38 and vacuum pump motor 40. The container 12 has an inlet 14 for air and vapour entry and an outlet 16 for air and vapour withdrawal.

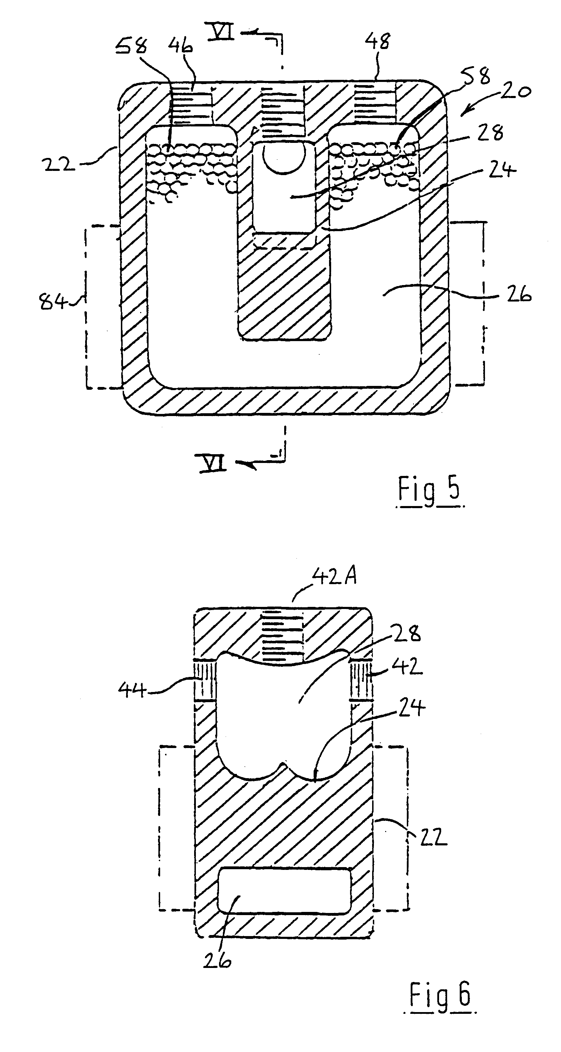

The vaporiser and degrader may be two separate units appropriately connected within the system. In the preferred embodiment, however, they are used together as a single unit. The vaporiser/degrader 20 is shown in greater detail in Figs. 5 and 6. Vaporiser/degrader 20 has an external housing 22 and an internal housing 24. An outer chamber 26 is defined therebetween. The internal housing 24 defines an inner chamber 28. The outer chamber 26, which functions as the converter, houses a plurality of spheres 58 which define a low flow-resistant tortous path having a high surface area for degradation of the sterilant. When the system is used as a hydrogen peroxide sterilisation system, the metallic spheres 58 are preferably coated, or made entirely of, copper or some other material known to cataltyically degrade hydrogen peroxide. A heating element 84, such as an electric band heater, establishes within the outer chamber 26, temperatures sufficiently high in conjunction with the highly catalytic, high surface area tortuous pathway created by spheres 58, for the almost instantaneous catalytic decomposition of the sterilant vapour. Although the spheres 58 have been demonstrated to work well in decomposing hydrogen peroxide into water and oxygen, any suitable environment which completely degrades a particular sterilant to a form suitable for disposal will suffice.

Heat from heater 84 and, to some extent, the heat given off during the decomposition of the sterilant, is conducted through the spheres 58 and the internal housing 24 to inner chamber 28, which functions as the vaporiser to vaporise instantaneously the liquid sterilant when it enters the inner chamber 28 of vaporiser/degrader 20. The vaporiser temperature when used for hydrogen peroxide sterilisation is about 60 to 150°C (140 to 302°F). The vapour is then passed into the container 12 as described more fully below. The vapour may pass into the container 12 continually or may pass incrementally as disclosed in US- A- 4,642,165.

Vacuum pump motor 40 and the vacuum pump can be any suitable known motor and pump. The fluid connections between the components of the system may be any suitable known conduits, piping or similar connecting means.

In operation, vacuum motor 40 is turned on. Container 12 should be sealed. Vacuum pump driven by the motor 40 draws air from the container 12. The vacuum level within container 12 is preferably kept low by vacuum motor 40 to about one to ten inches water volume (249 to 2490 Pa) which is about 0.036 to 0.36 psi. As the pressure within container 12 decreases, vacuum motor 40 ultimately draws air from air intake 30 through flow meter 32 and air filter 34 along a suitable path 60 through port 42 into the inner chamber 28 of vaporiser/degrader 20 where the air may be optionally heated, then is drawn out through port 44 along path 62 and into container 12 through inlet 14. The change in temperature of the air stream upon passage through chamber 28 depends on the air flow rate and the temperature differential between the incoming air and the chamber surfaces. As the vacuum motor 40 continues to operate, the air drawn through vaporizer/degrader 20 into container 12 flows through container 12 and out through outlet 16 along path 64 past vacuum motor 40.

The air stream is preferably split between paths 66 and 68. Path 66 is preferably narrower than path 68 so that a substantial portion of the stream of air flows along path 68 to port 46 into outer chamber 26 of vaporiser/degrader 20 and out through port 48 to exhaust. A remaining portion of air flows along path 66 and is sufficient having regard to the reduced pressure in chamber 28 to create a pressure gradient across reservoir 36 to force liquid sterilant through path 70. In an alternative embodiment, path 66 may be eliminated. Suction from container 12, through vaporiser/degrader 20 draws liquid into chamber 28, as described below.

If injection valve 38 is open, liquid sterilant will flow along path 72 through port 42A into chamber 28 of vaporiser/degrader 20 where the liquid sterilant will be vaporised upon contact with the heated surface of inner chamber 28. The resulting vapour is then carried by the air stream created by vacuum motor 40 through port 44 along path 62 through inlet 14 into container 12. Any contamination introduced by the air itself which is not removed by air filter 34 will be sterilised upon exposure to the sterilant vapour.

The flow of vapour phase sterilant through container 12 continues for a time period suitable for sterilising the interior of container 12 and/or its contents. The system illustrated employs suitable known sterilisation cycles. Depending on the container contents, the simultaneous flow of air may create sufficient turbulence to mix the vapour phase sterilant throughout container 12. Additional or alternative means for creating turbulence, such as fans, may be provided. In the preferred embodiment, wherein the container 12 is an incubator, approximately 6.5 ft³ (184 e) in volume, and the sterilant is hydrogen peroxide, the rate of flow for hydrogen peroxide sterilisation is about 2.5 ft³/min (71ℓ/min). The optimum rate of flow may vary depending on the size of the container and the cycle time for effective sterilisation.

The vapour phase sterilant is withdrawn from container 12 through outlet 16 along paths 64 and 68 through port 46 into outer chamber 26 where, by virtue of the catalytic effect of spheres 58, the sterilant is degraded and exhausted through port 48. When vapour phase hydrogen peroxide is used as the sterilant, the degraded components are harmless and can be vented to the atmosphere. When other gases, such as perhaps ethylene oxide or formaldehyde are used as the sterilant, and adequate means for destroying harmful gases do not fit within outer chamber 26, the exhaust must be contained and treated according to suitable methods.

When no further sterilant entry into container 12 is required, the injection valve 38 is closed. Vacuum motor 50 continues for a period sufficient to permit the air stream to carry residual sterilant from container 12 to outer chamber 26 for degradation.

The illustrated sterilisation system is fail-safe in that it will not permit injection of liquid sterilant into vaporiser/degrader 20 when the container 12 is opened because the pressure differential across the reservoir 36 would be insufficient to urge the liquid from reservoir 36 into chamber 28 to vaporiser/degrader 20. With the door open, no suction would be created to draw vapour to chamber 28 and there would be no air flow through chamber 28 to carry the vapour into container 12. Furthermore, the slight suction within container 12 prevents container 12 from being opened during operation. Complicated interlocking systems for container 12 can be avoided. Some prior art systems, in contrast, placed a blower on the upstream side of the sterilisation chamber to push the vapour into the chamber. Such systems require interlocks on the chamber doors.

The slight suction created by vacuum motor 40 is substantially different from the degree of vacuum found in conventional vacuum sterilisation systems. Vacuum motor 40, therefore, can be smaller than the vacuum pumps required to pull a significant vacuum within conventional vacuum sterilizers. In addition, due to the pressure gradient across reservoir 36, a pump is not needed to meter the flow of sterilant through injection valve 38. A relatively inexpensive injection valve 38 can, therefore, be used in the system. Any suitable known injection valve 38 for selectively metering and controlling the flow of liquid will suffice. The flow-through system of the present invention can provide a relatively low cost module for use with a variety of containers not heretofore suitable as sterilisation chambers. The fluid connections of the module can be releasably attached to a container at an inlet and outlet of the container and sealed by suitable known connecting and sealing means, such as pipes, washers, gaskets, clamps and similar known devices.

An optional feature, shown in Fig. 1, is parallel path 61 and flow restrictor 65. Parallel path 61 permits some of the air from air intake 30 to bypass chamber 28. The remainder of the air flows through chamber 28. The air diverted along parallel path 61 joins the air of air/vapour mixture exiting chamber 28 and cools it. Flow restrictor 65, which is preferably a drilled orifice in path 60 having a smaller diameter than the diameter of path 61, can be used to divert most of the air along path 61. Alternatively, path 61 may have a flow restrictor so that most of the air enters chamber 28. The temperature of the air or air/vapour mixture entering container 12 can be controlled by adjusting the relative sizes of the orifices and paths 60, 61 and 65. Use of a parallel path 61 to cool air or the air/vapour mixture entering container 12 permits the use of greater heat in chamber 26 to provide a higher efficiency breakdown of sterilant vapour while still controlling the temperature of the air/vapour mixture entering the container 12.

An alternative embodiment of the sterilisation system of the present invention is shown in Fig. 2, wherein parts like those of Fig. 1 are denoted by like reference numerals. A second liquid reservoir 50 and injector valve 52 with appropriate pathways 80, 81 and 82 are added. In addition, a three-way diverter valve 54 is placed in path 68. An additional path 75 and a flow restrictor 56 lead from the diverter valve 54 to path 60 just before port 42 into inner chamber 28 of vaporiser/degrader 20.

The second reservoir 50 can hold water, for example, to humidify the air stream and container 12, if desired. When injector valve 52 is opened, injector valve 38 associated with reservoir 36 is closed. Similarly, when injector valve 38 is open, injector valve 52 is closed. The two liquid reservoirs, 36 and 50, are not ordinarily operated simultaneously, but, either may be operated whether diverter valve 54 is in position A + B or A + C.

When diverter valve 54 is open from A + B along path 68, the valve path A + C to path 75 is closed and air flow is the same as in Fig. 1 as explained above. When A + C is open, the valve path A + B is closed. The path A + C through diverter valve 54 along path 75 is used when air and/or vapour is to be recirculated through container 12 and not exhausted. The flow restrictor 56, which may be merely a drilled orifice along path 75, creates a pressure differential in the system. Path 75, due to flow restrictor 56 and the vacuum pump driven by motor 40, is at a positive pressure relative to path 60 and container 12 is at a negative pressure relative to path 60. The air stream flows along path 75 towards port 42 into vaporiser/degrader 20. Back flow of air out of the air intake 30 is prevented by flow meter 32.

Humidification of container 12 is achieved by opening valve 52 and operating vacuum motor 40 as described above to urge water from reservoir 50 to chamber 28 where the water is vaporised and carried by an air stream through container 12. When sufficient water vapour has been formed in the system, valve 52 is closed. Diverter valve path A + C is open to direct the flow of water vapour and air withdrawn from container 12 through path 75 into chamber 28 for recirculation through container 12. When the water vapour needs to be replenished, valve 52 is opened for a suitable time. The pressure gradient created by flow restrictor 56 is sufficient, with the aid of vacuum motor 40, to recirculate the water vapour and air.

Aeration is achieved by closing valves 38 and 52 and opening path A + B of diverter valve 54, thereby supplying a continuous fresh stream of air to container 12. Alternatively, as illustrated in Fig. 2, a diverter valve 104 can be placed in the exhaust line leading from chamber 26. The air stream can pass through the converter to degrade any residual sterilant, then exit port 48 and pass back into container 12 through valve path A + B. The already sterilised air is thereby recirculated through the system.

When the container 12 is an incubator, the humid air flow offered by liquid reservoir 50 permits elimination of the water pan typically used to provide moisture for incubators. Water pans are known to provide a site for bacterial growth. In addition, the humidification provided by the flow-through system is significantly faster than the humidification obtained from use of a water pan which relies on natural evaporation. Furthermore, every time the incubator door is opened, the humidity within container 12 falls. Natural make up time can be about 8 to 12 hours. Humidification by the system of the present invention is about one-half hour.

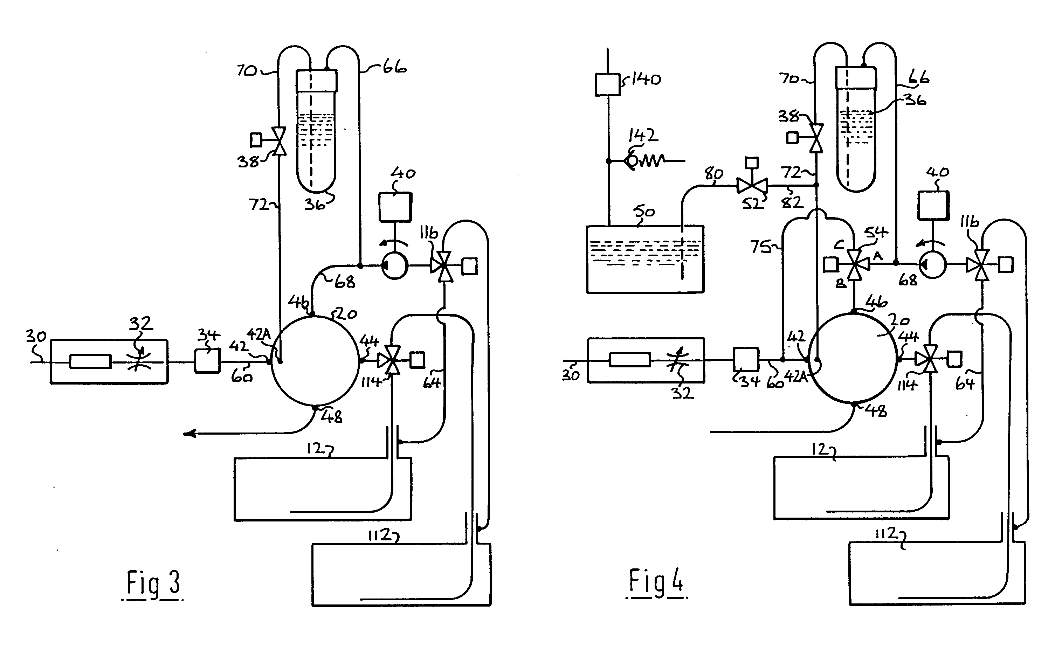

A third embodiment of the flow-through system of the present invention is illustrated schematically in Fig. 3, wherein parts like those of Fig. 1 are denoted by like reference numerals. A "time-share" system having dual sterilisation containers 12 and 112 is provided. Three-way diverter valves 114 and 116 of a known variety control the flow of air and vapour into and out of containers 12 and 112. This embodiment of the system operates in the same manner as described above with the exception of the additional selective operation of valves 114 and 116 required to control flow to and from the desired container.

A fourth embodiment, illustrated schematically in Fig. 4, combines the dual container system of Fig. 3 with the dual liquid reservoir system of Fig. 2, with the exception that an extra air pump 140 and a solenoid valve 142 of a known variety are added to force liquid from reservoir 50 towards vaporiser/degrader 20. Parts in Fig. 4 like those of Figs. 2 and 3 are denoted by like reference numerals. The embodiment of the system of Fig. 4 operates in the same fashion as the embodiments described above except that, when the second liquid reservoir 50 is used, valve 38 is closed and pump 140 and solenoid valve 142 are activated.

## Claims

1. A vapour flow-through system for use with at least one sealable pressure or non-pressure container (12,112) comprising air intake means (30); a fluid flow path (60,62) for connecting the air intake means (30) to an inlet (14) to the container (12); means (40) for withdrawing air from an outlet (16) from the container (12); at least one reservoir (36) for fluid; and heating means (20) in said fluid flow path (60,62), characterised in that the reservoir (36) contains a degradable liquid and a means (26,58) for degrading vapourized liquid to render it suitable for disposal is provided; and in that the reservoir (36) is connected via valve means (38) to the heating means (20) for conducting liquid thereto for heating such liquid to a temperature sufficient to transform the liquid into vapour substantially instantaneously and for introducing it into the air in the fluid flow path (60,62), whereby, in operation, a pressure differential is created between the reservoir (36) and the heating means (20) to urge liquid to flow from said reservoir (36) to said heating means (20) when said valve means (38) is open and withdrawal of air from the container (12) reduces the pressure therein sufficiently to draw a stream of air from said air intake means (30) through said heating means where said stream of air carries vapour formed in said heating means (20) through said container inlet (14) into said container (12), and whereby, in operation, a substantial portion of air and vapour withdrawn from said container (12) by said withdrawing means (40) is directed to said degrading means (26,58) for degradation and subsequent disposal.

2. A system as claimed in claim 1, which is a modular unit adapted for releasable attachment to said container (12).

3. A vapour flow-through system comprising at least one sealable pressure or non-pressure container (12,112); air intake means (30) connected via a fluid flow path (60,62) to an inlet (14) to the container (12); means (40) for withdrawing air from an outlet (16) from the container (12); at least one reservoir (36) for fluid; and heating means (20) in said fluid flow path (60,62), characterised in that the reservoir (36) contains a degradable liquid and a means (26,58) for degrading vapourized liquid to render it suitable for disposal is provided; and in that the reservoir (36) is connected via valve means (38) to the heating means (20) for conducting liquid thereto for heating such liquid to a temperature sufficient to transform the liquid into vapour substantially instantaneously and for introducing it into the air in the fluid flow path (60,62) and the degrading means (26,58) is in fluid communication with the container outlet (16), whereby, in operation, a pressure differential is created between the reservoir (36) and the heating means (20) to urge liquid to flow from said reservoir (36) to said heating means (20) when said valve means (38) is open and withdrawal of air from the container (12) reduces the pressure therein sufficiently to draw a stream of air from said air intake means (30) through said heating means where said stream of air carries vapour formed in said heating means (20) through said container inlet (14) into said container (12), and whereby a substantial portion of air and vapour withdrawn from said container (12) by said withdrawing means (40) is directed to said degrading means (26,58) for degradation and subsequent disposal.

4. A system as claimed in claim 3, wherein said container (12) is an integral part thereof.

5. A system as claimed in claim 3 or 4 wherein said sealable container (12) is an incubator.

6. A system as claimed in any of claims 1 to 5, further comprising a three-way valve (54) for selectively directing flow from said container (12) to said degrading means (26,58) for disposal or to said heating means (20) for recirculation.

7. A system as claimed in claim 6, further comprising means (56) for creating a pressure differential between said three-way valve (54) and said heating means (20) such that the pressure differential created thereby and said withdrawing means (40) are sufficient to draw flow from said three-way valve (54) to said heating means (20).

8. A system as claimed in any of claims 1 to 5, further comprising:
means (61) for diverting at least a portion of air flow around said heating means (20) and for merging such diverted air flow with fluids leaving said heating means (20) between said heating means (20) and said container (12) to reduce the temperature of such fluids.

9. A system as claimed in claim 8, wherein said diverting means is a bypass conduit (61) having an inlet side disposed between said air intake means (30) and said heating means (20) and an outlet side disposed downstream of said heating means (20), said system further comprising a flow restricter (65) to control the relative portions of air flow entering said bypass conduit (61) and said heating means (20).

10. A system as claimed in any of claims 1 to 9, further comprising an air filter (34) and an air flow meter (32) between said air intake means (30) and said heating means (20).

11. A system as claimed in any of claims 1 to 10, wherein there are two liquid reservoirs (36,50) each having associated therewith a respective valve means (38,52) for controlling delivery of liquid to said heating means (28) and each being adapted for fluid connection to said container (12).

12. A system as claimed in claim 11, wherein one liquid reservoir (36) contains a sterilant and the other liquid reservoir contains water for selectively providing a sterilisation system or a humidification system, respectively.

13. A system as claimed in any of claims 1 to 12, for use with two said containers (12,112) wherein said system further comprises:
an intake three-way valve (114) for disposition between said heating means (20) and said two containers (12,112) for selectively directing flow to one container (12) or to the other container (112); and
an outlet three-way valve (116) for disposition between said containers (12,112) and said withdrawing means (40) for selectively controlling flow from each container.

14. A system as claimed in claims 11 and 13, wherein one reservoir (50) has associated therewith pumping means (140) for exerting pressure within such reservoir (50) sufficient for urging liquid therefrom, when said associated valve means (52) is opened to said heating means (20).

15. A system as claimed in any of claims 1 to 14, wherein said heating means (20) and said converting means (26,58) are housed in a single unit comprising:
an inner housing (24) defining an inner chamber (28), said inner chamber having at least one first inlet and a first outlet;
an outer housing (22) surrounding said inner housing (24) to define therebetween an outer chamber (26), said outer chamber having a second inlet and a second outlet;
means (58) within said outer chamber (26) for degrading the vapour to a form suitable for disposal, said degrading means providing sufficient contact with incoming vapour to promote substantially complete degradation; and
a heater (84) for providing sufficient heat to said outer chamber (26) to enhance conversion of incoming vapour and for providing sufficient heat for said inner chamber (28) to substantially instantaneously transform incoming liquid into vapour.

16. A system as claimed in any of claims 1 to 15 wherein said reservoir (36) contains a sterilant liquid.

17. A system as claimed in any of claims 1 to 15, wherein said reservoir (36) contains a solution of hydrogen peroxide.

18. A system as claimed in claims 15 and 17 wherein said converting means is a plurality of metallic spheres (58) having outer surfaces made of material for catalytically degrading hydrogen peroxide vapour.

19. A system as claimed in claim 18, wherein the degradation of incoming vapour produces heat for transfer to said inner housing (24) to aid in heating incoming fluid.

20. A system as claimed in any of claims 1 to 19, wherein said path (64) is split after passing said withdrawing means (40) into a first connection (68) directed to said degrading means (26,58) and a second connection (66) directed to said reservoir (36).

21. Apparatus for use with a vapour system comprising:
an inner housing (24) defining an inner chamber (28) having at least one first inlet (42) and a first outlet (44);
an outer housing (22) surrounding said inner housing (24) to define therebetween an outer chamber (26) having a second inlet (46) and a second outlet (48);
means (58) within said outer chamber (26) for degrading a degradable vapour to a form suitable for disposal, said degrading means (58) providing sufficient contact with incoming vapour to promote substantially complete degradation; and
heating means (84) for imparting sufficient heat to said outer chamber (26) to enhance degradation of incoming vapour and for providing sufficient heat for said inner chamber (28) to substantially instantaneously transform incoming liquid into vapour.

22. Apparatus as claimed in claim 21, wherein said degrading means is a plurality of metallic spheres (58), at least the surfaces of which catalytically degrade the degradable vapour to a form suitable for disposal.

23. Apparatus as claimed in claim 22, wherein said heating means is an electric heater (84) associated with said outer housing (22) and heat from said electric heater and from the degradation of vapour is conducted through said metallic spheres to said inner housing (24).

## Patentansprüche

1. Vorrichtung zum Durchströmen von Dampf mit mindestens einem druckfreien Behälter oder Druckbehälter (12,112) bestehend aus einer Lufteinlaßvorrichtung (30); einem Flüssigkeitsdurchströmungskanal (60,62) zur Verbindung der Lufteinlaßvorrichtung (30) mit einem Eingang (14) zum Behälter (12); einer Vorrichtung (40) zum Abzug von Luft durch einen Ausgang (16) aus dem Behälter (12); mindestens einem Flüssigkeitsspeicher (36); und einer Erhitzungsvorrichtung (20) im besagten Flüssigkeitsdurchströmungskanal (60,62), dadurch gekennzeichnet, daß der Speicher (36) eine abbaubare Flüssigkeit enthält und eine Vorrichtung (26,58) zum Abbau von verdampfter entsorgungsfähiger Flüssigkeit eingebaut ist; und daß der Speicher (36) über eine Ventilvorrichtung (38) mit der Erhitzungsvorrichtung (20) verbunden ist, um Flüssigkeit zu dieser hinzuleiten, um diese Flüssigkeit auf eine Temperatur zu erhitzen, die ausreicht, um die Flüssigkeit sofort und vollständig in Dampf umzuwandeln und um ihn der Luft im Luftdurchströmungskanal (60,62) zuzuführen, wobei zwischen dem Speicher (36) und der Erhitzungsvorrichtung (38) ein Druckunterschied erzeugt wird, um die Flüssigkeit von dem besagten Speicher (36) zu der besagten Erhitzungsvorrichtung (20) durch die besagte Behältereinlaßstelle (14) in den besagten Behälter (12) zu lenken, und wobei ein beträchtlicher Teil der bzw. des aus dem besagten Behälter mit Hilfe der besagten Abzugsvorrichtung (40) abgezogenen Luft bzw. Dampfes zu der besagten Zersetzungsvorrichtung (26,58) zur Zersetzung und zur anschließenden Entsorgung geleitet wird.

2. Vorrichtung nach Anspruch 1, welche eine Moduleinheit darstellt, die an den besagten Behälter an- und abgebaut werden kann.

3. Vorrichtung zum Durchströmen von Dampf bestehend aus mindestens einem abdichtbaren Druckbehälter oder einem druckfreien Behälter (12,112); einer Lufteinlaßvorrichtung (30) über einen Flüssigkeitsdurchströmungskanal (60,62) mit einer Einlaßstelle (14) zum Behälter (12) verbunden; einer Vorrichtung (40) zum Abzug von Luft aus der Auslaßstelle (16) vom Behälter (12); mindestens einem Flüssigkeitsspeicher (36); einer Erhitzungsvorrichtung (20) in besagtem Flüssigkeitsdurchströmungskanal (60,62), dadurch gekennzeichnet, daß der Speicher (36) eine abbaubare Flüssigkeit enthält und eine Vorrichtung (26,58) zum Abbau von verdampfter, entsorgungsfähiger Flüssigkeit eingebaut ist; und daß der Speicher (36) über eine Ventilvorrichtung (38) mit der Erhitzungsvorrichtung (20) verbunden ist, um dieser Flüssigkeit zuzuleiten, die solange erhitzt wird, bis sie eine Temperatur erreicht hat, die die Flüssigkeit sofort und vollständig in Dampf umwandelt, um sie der Luft im Luftdurchströmungskanal (60,62) zuzuführen, und daß zwischen der Abbau/Zersetzungsvorrichtung (26,58) und der Auslaßstelle (16) des Behälters (12) eine Flüssigkeitsübertragung stattfindet, wobei zwischen dem Speicher (36) und der Erhitzungsvorrichtung (20) ein Druckunterschied erzeugt wird, um die Flüssigkeit vom besagten Speicher (36) zu der besagten Erhitzungsvorrichtung (20) zu leiten, wenn die besagte Ventilvorrichtung (38) geöffnet ist und Abzug von Luft aus dem Behälter (12) den darin enthaltenen Druck ausreichend verringert, damit ein Luftstrom aus der besagten Lufteinlaßstelle (30) durch die besagte Erhitzungsvorrichtung abgezogen wird, in der besagter Luftstrom in besagter Erhitzungsvorrichtung (20) gebildeten Dampf durch besagte Behältereinläßstelle (14) in besagten Behälter (12) geleitet wird, und wobei ein beträchtlicher aus besagtem Behälter (12) mit Hilfe besagter Abzugsvorrichtung (40) abgezogener Teil von Luft und Dampf in besagte Abbau/ Zersetzungsvorrichtung (26,58) zum Abbau/zur Zersetzung und zur anschließenden Entsorgung geleitet wird.

4. Vorrichtung nach Anspruch 3, worin besagter Behälter (12) ein wesentlicher Bestandteil hiervon darstellt.

5. Vorrichtung nach Anspruch 3 oder 4 , worin besagter Behälter (12) ein Inkubator ist.

6. Vorrichtung nach einem der Ansprüche von 1 bis 5, weiter bestehend aus einem Drei-Wege-Ventil (54) zur getrennten Weiterleitung des Stroms aus besagtem Behälter (12) zu besagter Abbau/Zersetzungsvorrichtung (26,58) zur Entsorgung oder zu besagter Erhitzungsvorrichtung (20) zur nochmaligen Zirkulation.

7. Vorrichtung nach Anspruch 6, zusätzlich bestehend aus einer Vorrichtung (56) zur Erzeugung eines Druckunterschiedes zwischen besagtem Drei-Wege-Ventil (54) und besagter Erhitzungsvorrichtung (20), so daß der dadurch erzeugte Druckunterschied und besagte Abzugsvorrichtung (40) ausreichen, um den Fluß aus besagtem Drei-Wege-Ventil (54) in besagte Erhitzungsvorrichtung (20) zu leiten.

8. Vorrichtung nach einem der Ansprüche von 1 bis 5, zusätzlich bestehend aus deiner Vorrichtung (61) zum Umleiten von mindestens einem Teil des Luftstromes um besagte Erhitzungsvorrichtung (20) und um diesen umgeleiteten Luftstrom und die aus besagter Erhitzungsvorrichtung (20) austretende Flüssigkeit zwischen besagter Erhitzungsvorrichtung (20) und besagtem Behälter (12) zusammenzuführen, um die Temperatur dieser Flüssigkeiten zu verringern.

9. Vorrichtung nach Anspruch 8, in der besagte Umleitungsvorrichtung ein Umleitungskanal (61) ist, versehen mit einer Einlaßseite angebracht zwischen besagter Lufteinlaßvorrichtung (30) und besagter Erhitzungsvorrichtung (20) und einer Auslaßseite angebracht stromabwärts an besagter Erhitzungsvorrichtung (20), in der besagte Vorrichtung zusätzlich besteht aus einem Durchströmungshemmer (65) zur Regulierung des relativen Teiles des in besagten Umleitungskanal (61) und besagte Erhitzungsvorrichtung (20) eintretenden Luftstromes.

10. Vorrichtung nach einem der Ansprüche von 1 bis 9, zusätzlich bestehend aus einem Luftfilter (34) und einem Luftstrommesser (32) zwischen besagter Lufteinlaßstelle (30) und besagter Erhitzungsvorrichtung (20).

11. Vorrichtung nach einem der Ansprüche von 1 bis 10, mit zwei Flüssigkeitsspeichern (36,50) mit jeweils einer dazugehörenden Ventilvorrichtung (38,52) zur Regulierung der Flüssigkeitszufuhr zu besagter Erhitzungsvorrichtung (28), wobei jeder zum Zweck der Flüssigkeitsverbindung mit besagtem Behälter (12) verbunden ist.

12. Vorrichtung nach Anspruch 11, in der ein Flüssigkeitsspeicher einen Sterilisator und der andere Flüssigkeitsspeicher Wasser enthält, um so zwei getrennte Vorrichtungen, eine zur Sterilisation und die andere zur Befeuchtung, zu bieten.

13. Vorrichtung nach einem der Ansprüche von 1 bis 12, zur Verwendung zusammen mit besagten Behältern (12,112), in der besagte Vorrichtung zusätzlich besteht aus:
einem Drei-Wege-Einlaßventil (114), welches sich zwischen besagter Erhitzungsvorrichtung (20) und besagten zwei Behältern (12,112), um den Strom getrennt in den einen (12) oder den anderen Behälter (112) zu leiten; und
einem Drei-Wege-Auslaßventil (116), welches sich zwischen besagten Behältern (12,112) und besagter Abzugsvorrichtung (40) zur getrennten Weiterleitung des Stromes aus besagten Behältern (12,112).

14. Vorrichtung nach Ansprüchen 11 und 13, in der sich ein Speicher (50) mit dazugehörender Pumpvorrichtung (140) befindet, um innerhalb des Speichers Druck zu erzeugen, welcher ausreicht, um bei besagter geöffneter Ventilvorrichtung (52) Flüssigkeit aus dem Speicher in besagte Erhitzungsvorrichtung (20) zu pumpen.

15. Vorrichtung nach einem der Ansprüche von 1 bis 14, in der sich besagte Erhitzungsvorrichtung (20) und besagte Abbau/Zersetzungsvorrichtung (26,58) in einer einzigen Einheit befinden, bestehend aus einem inneren Gehäuse (24), welches eine Innenkammer (28) abgrenzt, wobei gesagte Innenkammer mindestens eine erste Eintrittsstelle und eine erste Austrittsstelle aufweist;
einem äußeren Gehäuse (22), welches besagtes innere Gehäuse (24) umschließt, um so dazwischen eine Außenkammer (26) abzugrenzen, wobei besagte Außenkammer eine zweite Eintrittsstelle und eine zweite Austrittsstelle aufweist;
eine Vorrichtung (58) innerhalb besagter Außenkammer (26) zum Abbau von Dampf in eine entsorgungsfähige Form, wobei besagte Abbauvorrichtung genügend Kontakt mit eintretendem Dampf hat, um einen vollständigen Abbau zu fördern; und
einen Erhitzer (84) sowohl zur Erzeugung von ausreichender Hitze für die Außenkammer (26), um die Umwandlung des eintretenden Dampfes zu fördern als auch zur Erzeugung von ausreichender Hitze für die Innenkammer (28), um die eintretende Flüssigkeit sofort vollständig in Dampf umzuwandeln.

16. Vorrichtung nach einem der Ansprüche von 1 bis 15, in der besagter Speicher (36) eine Sterilisationsflüssigkeit enthält.

17. Vorrichtung nach einem der Ansprüche von 1 bis 15, in der besagter Speicher (36) eine Wasserstoff-Peroxid-Lösung enthält.

18. Vorrichtung nach Ansprüchen 15 und 17, in der besagte Abbauvorrichtung eine Vielzahl von kugelförmigen Körpern (58) ist, welche eine äußere Oberfläche bestehend aus einem Material zum katalytischen Abbau von Wasserstoff-Peroxid-Dampf aufweisen.

19. Vorrichtung nach Anspruch 18, in der der Abbau des eintretenden Dampfes Hitze erzeugt, welche zum inneren Gehäuse (24) geleitet wird, um somit die eintretende Flüssigkeit zu erhitzen.

20. Vorrichtung nach einem der Ansprüche von 1 bis 19, in der besagter Kanal (64) nach Durchgang durch besagte Abzugsvorrichtung (40) getrennt wird, in einen ersten Verbindungskanal (68), welcher in besagte Abbauvorrichtung (26,58) führt und in einen zweiten Verbindungskanal (66), welcher in besagten Speicher führt (36).

21. Ein Apparat zur Verwendung mit einer Dampf-Vorrichtung, bestehend aus:
einem inneren Gehäuse (24), welches eine Innenkammer (28) mit mindestens einer ersten Eintrittsstelle (42) und mindestens einer ersten Austrittsstelle (44) abgrenzt;
einem äußeren Gehäuse (22), welches besagtes innere Gehäuse (24) umschließt, um so dazwischen eine Außenkammer (26) mit einer zweiten Eintrittsstelle (46) und einer zweiten Austrittsstelle (48) abzugrenzen;
einer Vorrichtung (58) innerhalb besagter Außenkammer (26) zum Abbau von abbaubarem Dampf in eine entsorgungsfähige Form, wobei besagte Abbauvorrichtung (58) genügend Kontakt mit dem eintretenden Dampf hat, um einen vollständigen Abbau zu fördern; und
eine Erhitzungsvorrichtung (84), um sowohl genügend Hitze in besagte Außenkammer (26) einströmen zu lassen, um so den Abbau des eintretenden Dampfes zu fördern als auch genügend Hitze für besagte Innenkammer (28) zu liefern, um die eintretende Flüssigkeit sofort vollständig in Dampf umzuwandeln.

22. Ein Apparat nach Anspruch 21, in dem es sich bei der Abbauvorrichtung um eine Vielzahl von kugelförmigen Körpern (58) handelt, welche mindestens eine Oberfläche aufweisen, welche den abbaubaren Dampf in eine entsorgungsfähige Form abbauen.

23. Ein Apparat nach Anspruch 22, in dem es sich bei besagter Erhitzungsvorrichtung um ein elektrisches Heizgerät (84) handelt, welches mit besagtem äußeren Gehäuse (22) verbunden ist, und wobei Hitze sowohl aus besagtem elektrischen Heizgerät als auch aus dem Abbau von Dampf durch die metallischen kugelförmigen Körper zu besagtem inneren Gehäuse (24) geleitet wird.

## Revendications

1. Système de circulation de vapeur pour utilisation avec au moins un conteneur étanche sous pression ou non (12, 112) et comportant des moyens d'admission d'air (30); un passage d'écoulement de fluide (60, 62) afin de relier les moyens d'admission d'air (30) à une entrée (14) du conteneur (12); des moyens (40) destinés à extraire l'air par une sortie (16) du conteneur (12); au moins un réservoir (36) pour du fluide; et des moyens de chauffage (20) dans ledit passage d'écoulement de fluide (60, 62), caractérisé en ce que le réservoir (36) contient un liquide dégradable et des moyens (26, 58) destinés à dégrader le liquide vaporisé afin de le rendre adapté à une évacuation; et en ce que le réservoir (36) est relié à l'aide de moyens à soupape (38) aux moyens de chauffage (20) afin de conduire du liquide de façon à chauffer ce liquide à une température suffisante pour transformer le liquide en vapeur pratiquement instantanément et afin de l'introduire dans l'air dans le passage d'écoulement de fluide (60, 62), une différence de pression étant créée lors du fonctionnement entre le réservoir (36) et les moyens de chauffage (20) afin de forcer le liquide à s'écouler dudit réservoir vers lesdits moyens de chauffage (20) lorsque lesdits moyens de soupape (38) sont ouverts et l'évacuation de l'air du conteneur (12) réduisant suffisamment la pression pour aspirer un courant d'air par lesdits moyens d'admission d'air (30) à travers lesdits moyens de chauffage (20) où ledit courant d'air transporte de la vapeur formée dans lesdits moyens de chauffage (20) à travers ladite entrée de conteneur (14) dans ledit conteneur (12), et une partie substantielle de l'air et de la vapeur extraite dudit conteneur (12) par lesdits moyens d'extraction étant en fonctionnement dirigée vers lesdits moyens de dégradation (26, 58) pour dégradation et évacuation consécutive.

2. Système selon la revendication 1, qui est une unité modulaire prévue pour une fixation démontable sur ledit conteneur (12).

3. Système de circulation de vapeur comportant au moins un conteneur étanche sous pression ou non (12, 112); des moyens d'admission d'air (30) reliés par un passage d'écoulement de fluide (60, 62) à une entrée (14) du conteneur (12); des moyens (40) destinés à extraire l'air par une sortie (16) du conteneur (12); au moins un réservoir (36) pour du fluide; et des moyens de chauffage (20) dans ledit passage d'écoulement de fluide (60, 62), caractérisé en ce que le réservoir (36) contient un liquide dégradable et des moyens (26, 58) destinés à dégrader le liquide vaporisé afin de le rendre adapté à une évacuation; et en ce que le réservoir (36) est relié à l'aide de moyens à soupape (38) aux moyens de chauffage (20) afin de conduire du liquide de façon à chauffer ce liquide à une température suffisante pour transformer le liquide en vapeur pratiquement instantanément et afin de l'introduire dans l'air dans le passage d'écoulement de fluide (60, 62) et les moyens de dégradation (26, 58) sont en communication de fluide avec la sortie de conteneur (16), une différence de pression étant créée lors du fonctionnement entre le réservoir (36) et les moyens de chauffage (20) afin de forcer le liquide à s'écouler dudit réservoir vers lesdits moyens de chauffage (20) lorsque lesdits moyens de soupape (38) sont ouverts et l'évacuation de l'air du conteneur (12) réduisant suffisamment la pression pour aspirer un courant d'air par lesdits moyens d'admission d'air (30) à travers lesdits moyens de chauffage où ledit courant d'air transporte de la vapeur formée dans lesdits moyens de chauffage (20) à travers ladite entrée de conteneur (14) dans ledit conteneur (12), et une partie substantielle de l'air et de la vapeur extraite dudit conteneur (12) par lesdits moyens d'extraction (40) étant en fonctionnement dirigée vers lesdits moyens de dégradation (26, 58) pour dégradation et évacuation consécutive.

4. Système selon la revendication 3, dans lequel ledit conteneur (12) est une partie intégrante de celui-ci.

5. Système selon la revendication 3 ou 4, dans lequel ledit conteneur étanche (12) est un incubateur.

6. Système selon l'une quelconque des revendications 1 à 5, comportant en outre une soupape de détournement (54) destinée à diriger de manière sélective un écoulement dudit conteneur (12) vers lesdits moyens de dégradation (26, 58) pour évacuation ou vers lesdits moyens de chauffage (20) pour remise en circulation.

7. Système selon la revendication 6, comportant en outre des moyens (56) destinés à créer une différence de pression entre ladite soupape à trois voies (54) et lesdits moyens de chauffage (20) de telle sorte que la différence de pression ainsi crée et lesdits moyens d'évacuation (40) sont suffisants pour entraîner l'écoulement de ladite soupape à trois voies (54) vers lesdits moyens de chauffage (20).

8. Système selon l'une quelconque des revendications 1 à 5, comportant en outre : des moyens (61) destinés à détourner au moins une partie de l'écoulement d'air autour desdits moyens de chauffage (20) et afin de mélanger ledit écoulement d'air détourné à des fluides quittant lesdits moyens de chauffage (20) entre lesdits moyens de chauffage (20) et ledit conteneur (12) de façon à réduire la température de ces fluides.

9. Système selon la revendication 8, dans lequel lesdits moyens de détournement sont constitués par une conduite de dérivation (61) ayant un côté d'entrée disposé entre lesdits moyens d'admission d'air (30) et lesdits moyens de chauffage (20) et un côté de sortie disposé en aval desdits moyens de chauffage (20), ledit système comportant en outre un limiteur d'écoulement (65) destiné à commander les parties relatives d'écoulement d'air entrant dans ladite conduite de dérivation (61) et lesdits moyens de chauffage (20).

10. Système selon l'une quelconque des revendications 1 à 9, comportant en outre un filtre à air (34) et un débitmètre d'air (32) entre lesdits moyens d'admission d'air (30) et lesdits moyens de chauffage (20).

11. Système selon l'une quelconque des revendications 1 à 10, dans lequel il y a deux réservoirs de liquide (36, 50) ayant chacun des moyens de soupape respectifs associés (38, 52) afin de commander la sortie de liquide vers lesdits moyens de chauffage (28) et qui sont chacun prévus pour un raccordement de fluide audit conteneur (12).

12. Système selon la revendication 11, dans lequel un réservoir de liquide (36) contient un agent de stérilisation et l'autre réservoir de liquide contient de l'eau afin de procurer de manière sélective respectivement un système de stérilisation ou un système d'humidification.

13. Système selon l'une quelconque des revendications 1 à 12, destiné à être utilisé avec deux conteneurs (12, 112), dans lequel ledit système comporte en outre :
une soupape d'admission à trois voies (114) destinée à être disposée entre lesdits moyens de chauffage (20) et lesdits conteneurs (12, 112) afin de diriger de manière sélective l'écoulement vers un conteneur (12) ou l'autre conteneur (112); et
une soupape de sortie à trois voies (116) destinée à être disposée entre lesdits conteneurs (12, 112) et lesdits moyens d'évacuation (40) afin de commander de manière sélective l'écoulement depuis chaque conteneur.

14. Système selon l'une quelconque des revendications 11 et 13, dans lequel un réservoir (50) a des moyens de pompage d'air associés (140) afin d'exercer une pression dans ce réservoir (50) qui est suffisante pour chasser le liquide de celui-ci, lorsque lesdits moyens de soupape associés (52) sont ouverts sur lesdits moyens de chauffage (20).

15. Système selon l'une quelconque des revendications 1 à 14, dans lequel lesdits moyens de chauffage (20) et lesdits moyens de conversion (26, 58) sont logés dans une unique unité comportant :
un boîtier interne (24) définissant une chambre interne (28), ladite chambre interne ayant au moins une première entrée et une première sortie;
un boîtier externe (22) entourant ledit boitier interne (24) afin de définir entre eux une chambre externe (26), ladite chambre externe ayant une deuxième entrée et une deuxième sortie;
des moyens (58) à l'intérieur de ladite chambre externe (26) afin de dégrader une vapeur dégradable dans une forme appropriée à l'évacuation, lesdits moyens de dégradation procurant un contact suffisant avec la vapeur qui entre afin de faciliter une dégradation pratiquement complète; et
un dispositif de chauffage (84) destiné à apporter une chaleur suffisante à ladite chambre externe (26) afin d'améliorer la conversion de la vapeur qui entre et afin de procurer une chaleur suffisante à ladite chambre interne (28) afin de transformer pratiquement instantanément le liquide qui entre en vapeur.

16. Système selon l'une quelconque des revendications 1 à 15, dans lequel ledit réservoir de liquide (36) contient un agent de stérilisation liquide.

17. Système selon l'une quelconque des revendications 1 à 15, dans lequel ledit réservoir de liquide (36) contient une solution de peroxyde d'hydrogène.

18. Système selon l'une quelconque des revendications 15 et 17, dans lequel lesdits moyens de conversion sont constitués par plusieurs sphères métalliques (58) ayant des surfaces extérieures réalisées dans une matière destinée à dégrader de manière catalytique la vapeur de peroxyde d'hydrogène.

19. Système selon la revendication 18, dans lequel la dégradation de la vapeur qui entre produit de la chaleur pour transfert vers ledit boîtier interne (24) afin d'aider à chauffer le fluide qui entre.

20. Système selon l'une quelconque des revendications 1 à 19, dans lequel ledit passage (64) est séparé après être passé par lesdits moyens d'évacuation (40) en un passage (68) dirigé vers lesdits moyens de dégradation (26, 58) et une deuxième connexion (66) dirigée vers ledit réservoir (36).

21. Appareil pour utilisation avec un système de vapeur comportant :
un boîtier interne (24) définissant une chambre interne (28) ayant au moins une première entrée (42) et une première sortie (44);
un boîtier externe (22) entourant ledit boîtier interne (24) afin de définir entre eux une chambre externe (26) ayant une deuxième entrée (46) et une deuxième sortie (48);
des moyens (58) à l'intérieur de ladite chambre externe (26) afin de dégrader une vapeur dégradable dans une forme appropriée à l'évacuation, lesdits moyens de dégradation (58) procurant un contact suffisant avec la vapeur qui entre afin de faciliter une dégradation pratiquement complète; et
des moyens de chauffage (84) destinés à apporter une chaleur suffisante à ladite chambre externe (26) afin d'améliorer la dégradation de la vapeur qui entre et afin de procurer une chaleur suffisante à ladite chambre interne (28) afin de transformer pratiquement instantanément le liquide qui entre en vapeur.

22. Appareil selon la revendication 21, dans lequel lesdits moyens de dégradation sont constitués par plusieurs sphères métalliques (58), dont les surfaces au moins dégradent de manière catalytique la vapeur dégradable en une forme adaptée à l'évacuation.

23. Appareil selon la revendication 22, dans lequel lesdits moyens de chauffage (20) sont constitués par un dispositif de chauffage électrique (84) associé audit boîtier externe (22) et la chaleur provenant dudit dispositif de chauffage électrique et de la dégradation de la vapeur est conduite à travers lesdites sphères métalliques jusqu'audit boîtier interne (24) (24).
